# FASCICULE DE BREVET EUROPEEN

(11) **EP 2 493 626 B1**
(45) Date de publication et mention de la délivrance du brevet: **04.12.2013**
(21) Numéro de dépôt: 10787827.4
(22) Date de dépôt: 22.10.2010
(51) Int. Cl.: B05B 11/04, B65D 47/32, A61J 1/14, B65D 81/24, B65D 47/18, A61F 9/00, A61M 15/08, B05B 1/30, B05B 11/00

(54) **DISPOSITIF DE DISTRIBUTION DE LIQUIDE SOUS FORME DE GOUTTES**
VORRICHTUNG ZUR AUSGABE EINER FLÜSSIGKEIT IN TROPFENFORM
DEVICE FOR DISPENSING LIQUID IN THE FORM OF DROPS

(30) Priorité: 29.10.2009 FR 0957640
(43) Date de publication de la demande: 05.09.2012
(73) Titulaire: Rexam Healthcare La Verpillière, 38290 La Verpillière (FR)
(72) Inventeur: PAINCHAUD, Gaetan, F-69340 Francheville (FR); GREVIN, Guillaume, F-38080 L'Isle d'Abeau (FR); JULIA, Xavier, F-38090 Villefontaine (FR); DECOCK, Thierry, F-75013 Paris (FR); RIMLINGER, Thierry, F-38080 L'Isle d'Abeau (FR)
(74) Mandataire: Thiollier, Clémence-Olivia Laure Marie
(86) Numéro de dépôt international: PCT/FR2010/052258
(87) Numéro de publication internationale: WO 2011/051602

(56) Documents cités:
- WO-A1-93/22360
- US-A- 4 334 500
- US-A- 5 373 972

## Description

La présente invention concerne le domaine de la distribution de liquide, notamment sous forme dé gouttes dans le domaine pharmaceutique, par exemple du liquide ophtalmique.

Plus particulièrement, l'invention concerne la distribution de liquide sans conservateur, sous forme de gouttes, en utilisant un réservoir déformable avec reprise d'air.

La tendance actuelle est de fournir des produits, notamment ophtalmiques, sans conservateur. Il faut donc garantir la stérilité du produit tout au long de l'utilisation du flacon contenant le liquide à délivrer.

On connait, notamment des documents US 5,373,972 et WO92/01625, différents dispositifs qui permettent de délivrer des gouttes de produit contenus dans un réservoir et qui évitent la contamination du liquide restant dans le flacon.

Selon un exemple, un tel dispositif de distribution de liquide comprend un réservoir et un embout de distribution monté sur le réservoir, muni d'un orifice de distribution du liquide. L'utilisateur applique une pression sur le réservoir en le déformant et, sous l'effet de la pression, une goutte se forme à la surface de l'orifice de distribution. Une fois la goutte délivrée, l'utilisateur relâche la pression sur le réservoir déformable, lequel tend à reprendre sa forme initiale, ce qui génère une dépression à l'intérieur du flacon. Pour combler cette dépression et permettre au réservoir de reprendre sa forme initiale, l'embout du dispositif comprend une entrée d'air dans le réservoir. Pour assurer que l'air entrant ne puisse pas contaminer le liquide restant dans le réservoir, on prévoit, dans le passage d'air, un filtre hydrophobe. Ce filtre permet à l'air extérieur d'entrer dans le réservoir en évitant toute entrée de microorganismes et de poussières, tout en empêchant toute entrée ou sortie du liquide.

Un problème de ce type de dispositif réside dans le fait qu'il est délicat de garantir sa fiabilité. En effet, il est difficile de tester le bon fonctionnement du filtre après montage sur l'embout, car le filtre devrait alors être testé sous eau, ce qui implique un risque de contamination ou de dégradation lors de la phase de test. Il est donc difficile de garantir l'intégrité des filtres utilisés et de leur assemblage sur l'embout.

La présente invention a notamment pour but de proposer un dispositif de distribution de liquide garantissant de façon fiable la stérilité de l'embout de distribution.

A cet effet, l'invention a notamment pour objet un dispositif de distribution de liquide, caractérisé en ce qu'il comporte :
- un réservoir de liquide, déformable de façon à distribuer du liquide par appui sur le réservoir,
- un embout de distribution du liquide monté sur le réservoir,
- un canal de passage de liquide,
- un canal de passage d'air depuis l'extérieur vers l'intérieur du réservoir, le canal de passage d'air étant obturé par un organe réalisé en matériau polymère perméable à l'air, ce matériau étant non poreux, l'organe étant appelé organe perméable à l'air.

On propose donc de réaliser la fonction de reprise d'air non contaminé dans le réservoir, non pas en procédant à une filtration de l'air mais en utilisant les propriétés de diffusion de gaz de certains matériaux. Ainsi, on utilise un autre type d'organe qu'un filtre, à savoir un organe en matériau polymère non poreux. Un tel organe présente l'avantage d'assurer le passage d'air non contaminé de façon plus fiable qu'un filtre, lequel est poreux par définition. En effet, avec un organe non poreux, il n'est pas nécessaire de tester la taille de pores et il est plus facile de tester qu'il n'y a pas de fuite due à un mauvais assemblage ou à un organe défectueux.

Par matériau « non poreux », on entend une matière pleine, sans trou, bloquant le passage de particules telles que des bactéries, par exemple bloquant la bactérie Brevundimonas Diminuta ayant une taille de l'ordre de 0,2 micromètres. Ce matériau non poreux se distingue d'un filtre, réalisé de façon à être poreux. En effet, le matériau non poreux proposé pour l'organe perméable à l'air est composé d'un polymère utilisé dans sa forme brute, ayant subi par exemple une simple injection ou compression, alors qu'un matériau poreux tel que celui d'un filtre est composé d'un polymère ayant subi en outre des étapes de génération de pores ou interstices, telles qu'un étirement sur la matière ou une addition de solvant chimique dans le polymère. Le matériau étant non poreux, il est étanche aux liquides et ne laisse pas passer de particules telles que des poussières ou des microorganismes. En revanche, ce matériau est perméable à l'air, du fait qu'il laisse passer des éléments de la taille d'une molécule. En d'autres termes, le matériau non poreux proposé ci-dessus a une perméabilité aux gaz qui laisse passer les molécules d'air, à travers un réseau réticulé de longues chaines moléculaires enchevêtrées. En d'autres mots, l'organe en matériau non poreux est configuré pour laisser passer l'air par diffusion de l'air au travers de l'organe perméable à l'air. On notera que, du fait que le matériau est non poreux, le passage d'air à travers l'organe est un processus qui prend plusieurs minutes, voire plusieurs heures, et non quelques secondes, comme c'est le cas pour un filtre. Par exemple, pour un dispositif ayant permis de délivrer 240 µl de liquide, la dépression est quasiment compensée, c'est-à-dire que la pression interne du flacon est sensiblement équivalente à la pression externe au bout de 12 heures seulement. Le retour à une pression de l'ordre de la pression externe peut paraître long, mais les inventeurs à l'origine de l'invention ont constaté que ce n'était pas vraiment gênant pour l'application à la distribution de gouttes.

Ainsi, lorsque l'utilisateur relâche la pression sur le réservoir, après l'avoir pressé pour délivrer une goutte de liquide, la dépression entre l'intérieur et l'extérieur du flacon est compensée, lentement, par le passage d'air extérieur à travers l'organe perméable à l'air

On notera que, comme cet organe ne comporte pas de pores, il n'y a pas de risque de colmatage dû à une accumulation de microorganismes et de poussières dans les pores. Par ailleurs, il n'y a pas non plus de pression capillaire de liquide, lequel s'oppose au rétablissement de l'équilibre des pressions entre l'intérieur et l'extérieur du réservoir lorsque le dispositif est en position « tête en bas » et que du liquide est alors en contact avec l'organe perméable à l'air. Ces deux phénomènes sont présents lorsque l'on utilise un filtre.

Ce type d'organe peut être testé de façon très facile. Ainsi, on peut tester tous les organes d'obturation après montage sur l'embout, sans contamination ou dégradation de l'organe. De tels tests sont plus avantageux que ceux effectués sur les filtres, risquant de les contaminer ou dégrader lors du test d'étanchéité, ou alors pour lesquels on ne peut effectuer que des tests statistiques, sur des échantillons détruits au cours du test, procurant une information relativement limitée.

Le test des organes peut être réalisé par exemple en appliquant une pression d'air d'un côté de l'organe et en mesurant la pression de l'autre côté après quelques secondes. Comme le processus qui permet le rééquilibrage des pressions de chaque côté de l'organe est un processus qui prend plusieurs minutes, voire plusieurs heures, et non quelques secondes, l'échelle de temps n'est pas la même que pour tester un filtre. Aussi, à l'échelle de la seconde, un organe non défectueux ne permettra pas de déceler de perte de pression, alors qu'on constatera une baisse de pression flagrante si l'organe est défectueux ou mal monté sur l'embout. Ce test permet ainsi d'identifier toutes les pièces défectueuses. On notera que l'organe perméable à l'air est de fabrication particulièrement aisée et économique. Il se distingue ainsi du filtre hydrophobe qui est particulièrement coûteux à réaliser, d'une part pour assurer une filtration fine, d'autre part pour assurer son intégrité.

Le dispositif de distribution peut en outre comporter l'une ou plusieurs des caractéristiques suivantes.
- L'organe perméable à l'air comprend en outre au moins un canal de passage de liquide. On peut ainsi prévoir un organe présentant une grande surface d'échange avec le réservoir.
- Le canal de passage de liquide est un canal de limitation de débit de liquide, débouchant sur le canal de passage de liquide. De ce fait, il est possible de limiter le débit du liquide sortant du réservoir et d'éviter de délivrer le liquide sous forme de jet si l'utilisateur exerce une pression trop importante sur le réservoir. Ainsi on profite de l'organe perméable à l'air pour assurer la fonction de limitation de débit, ce qui permet de simplifier le montage de l'embout, en diminuant le nombre de pièces à assembler. Selon un exemple, le canal de limitation de débit a un diamètre faible relativement au diamètre du canal de passage de liquide, ou encore le canal de limitation de débit présente des changements brusques de direction, permettant de diminuer la charge.
- L'embout et l'organe ont chacun un axe central, les deux axes étant confondus. Cela permet de faciliter les opérations de montage de l'organe sur l'embout. En effet, il est facile de centrer une pièce par rapport à une autre. Par ailleurs, en disposant l'organe perméable à l'air au centre du dispositif, on peut prévoir qu'il ait une surface relativement grande, par exemple couvrant tout le col du réservoir du dispositif, et puisse ainsi offrir une plus grande surface de passage d'air, pour que l'équilibre des pressions interne et externe se réalise au plus vite.
- L'organe comporte une paroi dite de passage d'air, munie d'une pluralité de reliefs, pour augmenter la surface de passage d'air. Par exemple, on peut prévoir une paroi ondulée, à section sinusoïdale, en créneau ou encore en zig-zag. Ainsi, on augmente la surface d'échange d'air entre l'intérieur et l'extérieur du réservoir sans pour autant augmenter considérablement l'encombrement de l'organe. En effet, le flux d'air pouvant passer à travers la paroi de l'organe par perméabilité est directement proportionnel à la surface d'échange et inversement proportionnel à l'épaisseur de la paroi de l'organe. Une grande surface d'échange et une épaisseur faible de paroi favorisent la reprise d'air. Selon la vitesse de reprise d'air souhaitée, on pourra donc facilement modifier la géométrie de la paroi de l'organe en faisant varier les paramètres de surface d'échange et d'épaisseur. On comprend que les reliefs ménagés sur la paroi se distinguent de nervures, ils sont réalisés en parallèle sur les deux faces de la paroi de l'organe, l'épaisseur de la paroi étant sensiblement constante le long des reliefs et suffisamment faible pour autoriser le passage d'air, de façon à augmenter la surface d'échange de l'organe perméable à l'air.
- L'organe comporte des nervures de renfort. Ces nervures permettent de rigidifier l'organe. On comprend que de telles nervures correspondent à des augmentations locales de l'épaisseur de la paroi de l'organe, pour la rigidifier, formant ainsi des saillies sur une seule des deux faces de la paroi. Ces nervures se distinguent donc des reliefs décrits ci-dessus qui ont pour fonction d'augmenter la surface d'échange.
- L'organe a une forme générale cylindrique ou conique, dont la base comprend une collerette de fixation sur l'embout. Cette collerette présente de préférence une épaisseur de matière supérieure à l'épaisseur de la paroi de passage d'air, de façon qu'elle présente une certaine rigidité permettant d'assurer la fixation de l'organe, par exemple par serrage mécanique. Éventuellement, la collerette peut comporter des moyens de fixation mécanique, qui coopèrent avec des moyens portés par l'embout, par exemple par encliquetage. Grâce à la collerette, l'organe se place facilement sur l'embout et ne nécessite pas de moyens complexes de fixation. Par ailleurs, pour un même embout, il est simple, selon les caractéristiques de reprise d'air voulues, de proposer un organe dont la paroi de passage d'air peut présenter différents types de configurations, tout en ayant une collerette standard s'adaptant à l'embout.
- Le canal de passage de liquide est délimité par une surface annulaire extérieure de l'organe perméable à l'air. Ainsi, l'organe perméable à l'air n'est percé d'aucun orifice permettant le passage de liquide ce qui permet de dissocier le passage d'air et le passage de liquide.
- Le matériau polymère comprend un matériau élastomère, L'organe étant déformable, il peut éventuellement être monté sur l'embout par déformation légère de l'organe. Une fois mis en place, l'organe peut reprendre sa forme initiale et être fixé par serrage mécanique dans l'embout ce qui simplifie sa mise en place. De plus, la souplesse de l'élastomère permet d'éviter plus facilement des jeux entre l'organe et l'embout, par adaptation des surfaces de contact de l'organe avec les parois de l'embout.
- Le matériau polymère comprend du silicone (également appelé polysiloxane, composé inorganique formé d'une chaîne silicium-oxygène). La perméabilité aux gaz du silicone permet de favoriser davantage le processus de reprise d'air et d'en réduire le temps. De plus, le silicone présente l'avantage d'être inertes par rapport aux liquides pharmaceutiques.
- La distribution de liquide est contrôlée par une unique valve, pouvant prendre une configuration de blocage de liquide et une configuration de distribution de liquide. Ainsi, le dispositif se distingue d'un dispositif de distribution muni d'une pompe, dispositif dans lequel la déformation du réservoir n'est pas nécessaire pour permettre la délivrance du liquide.
- Le dispositif comporte une valve et un support comprenant une surface d'appui de la valve pour bloquer le passage de liquide, le support comprenant le canal de passage d'air et l'organe perméable à l'air étant rapporté sur le support. On obtient ainsi un dispositif particulièrement compact.
- Le temps d'équilibrage des pressions entre la pression interne du réservoir et la pression externe après la distribution de liquide est supérieur à 30 minutes, de préférence 1 heure. Certes, la reprise d'air se fait à travers un organe qui ne permet pas au réservoir de reprendre sa configuration initiale en un temps quasi instantané, mais en contrepartie, le dispositif proposé permet de garantir la non contamination de l'air venant de l'extérieur. On notera que ce temps de retour à la configuration initiale est supérieur à 30 minutes voire 1 heure même dans le cas où l'organe d'étanchéité est utilisé dans des conditions optimales, en étant complètement dégagé. En d'autres termes, même lorsque le dispositif n'est pas utilisé tête en bas (auquel cas de l'eau est disposée contre l'organe) et lorsqu'il ne présente aucune impureté, le temps de diffusion du gaz est relativement long, à la différence d'une filtration par un filtre, auquel cas ce temps est quasi instantané ou du moins de l'ordre de la seconde.

L'invention a également pour objet un lot de deux dispositifs tels que décrits ci-dessus, comportant deux embouts identiques, munis d'organes perméables à l'air ayant chacun une configuration différente. Par exemple, les organes ont une épaisseur ou une forme différentes.

L'invention sera mieux comprise à la lecture de la description qui va suivre, donnée uniquement à titre d'exemple et faite en se référant aux dessins dans lesquels :
- la figure 1 présente un dispositif selon l'invention ; et
- les figures 2a à d présentent des variantes d'un organe perméable à l'air du dispositif de la figure 1.

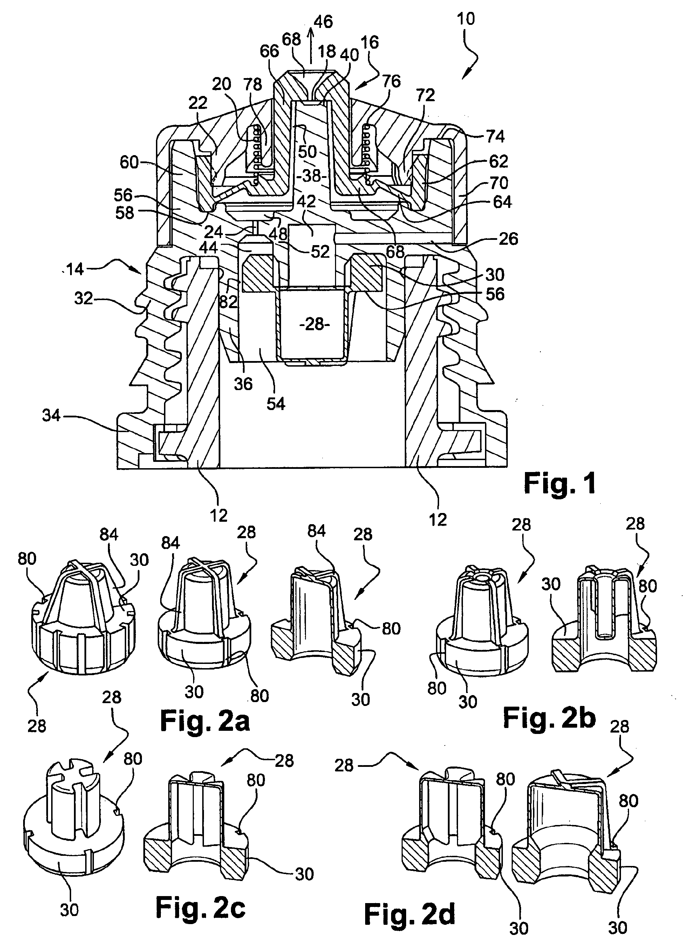

On a représenté sur à la figure 1 un embout 10 de distribution de liquide sous forme de goutte, destiné à être monté par vissage sur le col d'un réservoir 12. Ce réservoir 12 est un réservoir de stockage de liquide, par exemple du liquide pharmaceutique tel que du liquide ophtalmique. Le réservoir 12 est déformable de façon à distribuer du liquide par appui sur le réservoir. Plus précisément, la distribution de liquide se fait par pression, de la part d'un utilisateur, sur le corps du réservoir 12, ce dernier pouvant présenter une certaine élasticité pour reprendre sa forme initiale après la pression exercée par l'utilisateur ce qui génère une dépression à l'intérieur du réservoir 12.

L'embout de distribution 10 comprend, dans cet exemple, un support 14, une valve de distribution 16 munie d'un orifice de distribution 18, un ressort 20, un capot 22, un canal de passage de liquide 24 du réservoir 12 vers l'orifice de distribution 18 et un canal 26 de passage d'air dans le réservoir 12, canal 26 obturé par un organe 28 perméable à l'air.

Le support 14 comprend, dans cet exemple, une partie 32 de fixation sur le réservoir, disposée à l'extrémité proximale du support 14. Cette partie 32 comprend une jupe externe 34, comportant un filet de vissage de façon à être vissée sur le col du réservoir 12. La partie de fixation 32 comporte en outre une jupe interne 36 de forme tubulaire, permettant d'assurer l'étanchéité entre le réservoir 12 et l'embout de distribution 10.

Le support 14 comporte par ailleurs une partie centrale d'étanchéité 38, de forme sensiblement cylindrique et s'étendant dans la direction distale, opposée à la jupe interne 36. La partie 38 porte, sur son extrémité distale, une surface 40 d'appui de la valve 16 pour bloquer le passage de liquide en configuration de blocage. Dans cet exemple, la surface d'appui 40 prend la forme d'un bourrelet annulaire.

Le support 14 comporte également, dans cet exemple, le canal 26 de passage d'air dans le réservoir 12, qui débouche sur une cavité 42 de forme sensiblement cylindrique. Cette cavité 42 débouche, à son extrémité proximale, sur l'organe 28.

Le support 14 comprend également dans cet exemple un logement 44 formant une cavité de forme sensiblement cylindrique, cette cavité débouchant à son extrémité proximale sur le réservoir 12 et à son extrémité distale sur le canal 24 de passage de liquide, ménagé dans le support 14 et s'étendant dans la direction longitudinale du dispositif, correspondant ici à la direction d'éjection du liquide illustrée par la flèche 46. Le canal 24 débouche quant-à-lui sur une cavité intermédiaire 48, débouchant elle-même sur un deuxième canal 50 de passage de liquide.

Le logement 44 est juxtaposé à la cavité 42 en étant séparé par une paroi annulaire 52, s'étendant dans une direction opposée à la partie d'étanchéité 38.

L'organe 28 perméable à l'air est réalisé en matériau polymère perméable à l'air, ce matériau étant non poreux, ne laissant pas passer de particules telles que des bactéries de 0,1 micromètres, mais laissant passer des molécules, telles que des molécules de l'air. Ainsi, le passage d'air à travers l'organe 28 perméable à l'air est réalisé par diffusion de l'air au travers de l'organe 28. Le matériau polymère comprend un matériau élastomère, à savoir, dans cet exemple, du silicone. L'organe 28 a une forme générale cylindrique ou conique, il présente un axe central, confondu avec l'axe central de l'embout 10, cet axe correspondant à la direction de distribution de liquide, donc à la flèche 46. Plus précisément, l'organe 28 comprend dans cet exemple une paroi dite de passage d'air, d'épaisseur relativement fine afin de favoriser l'échange de gaz, de forme cylindrique ou conique, présentant un sommet obturé par une surface en forme de disque et une base comprenant une collerette annulaire 30 de fixation sur l'embout 10, cette collerette 30 ayant une épaisseur relativement importante, du moins supérieure à l'épaisseur générale de la paroi de passage d'air.

L'organe 28 se loge dans une cavité 54 de forme sensiblement cylindrique délimitée par la jupe interne 36 du support 14 et se fixe, dans cet exemple par serrage mécanique, par coopération de la collerette 30 avec la paroi annulaire 52. Plus précisément, le diamètre intérieur de la collerette 30 est légèrement inférieur au diamètre extérieur de la paroi 52, de telle sorte que la collerette est fixée sur la paroi 52 par élasticité. Eventuellement, on peut prévoir en outre des moyens de fixation mécanique de la collerette 30 sur la paroi 52, par exemple des moyens d'encliquetage tels qu'un bourrelet annulaire intérieur prévu sur la collerette 30 s'encliquetant dans une rainure annulaire prévue sur la surface extérieure de la paroi 52. On peut également imaginer des moyens d'accroche mécanique traversant la pièce 14 jusqu'à atteindre la cavité 48 ou alors des moyens d'accroche sur la paroi interne du cylindre 36.

Par ailleurs, le support 14 comprend une partie 56 de fixation de la valve 16 sur le support 14. Cette partie 56 fait également office de partie de fixation du capot 22 sur le support 14. Elle comprend une gorge annulaire 58 délimitée en périphérie par une paroi annulaire 60. La gorge annulaire 58 est par ailleurs délimitée, sur sa périphérie intérieure, par une nervure annulaire réalisée sur une paroi formant sensiblement un disque, traversée par le canal 24 et délimitant la cavité 48.

La valve 16 peut prendre une configuration de blocage et une configuration de passage du liquide, par coopération avec le support 14. Elle est réalisée, dans cet exemple, dans un matériau élastomère. Selon un autre exemple, seule une partie de la valve 16 est réalisée dans un matériau élastomère, l'autre partie étant réalisée dans un matériau plus rigide, pouvant servir d'appui au ressort 20. La valve 16 comprend une partie 62 de fixation au support 14, formant une jupe de forme sensiblement tubulaire. Cette partie de fixation 62 est reliée à un voile 64 ayant sensiblement la forme d'un disque et à partir duquel une partie centrale 66 sensiblement cylindrique fait saillie. Le voile 64 comporte également un siège 68 d'appui du ressort 20. La partie 66 définit une cavité intérieure de forme sensiblement cylindrique, complémentaire de la partie 38. La partie 38 et la partie cylindrique 66 sont coaxiales et délimitent ensemble le canal de passage 50. Ce canal de passage 50 débouche sur l'orifice de distribution 18 réalisé dans l'extrémité distale de la valve 16, débouchant lui-même sur une forme 68 de formation de gouttes.

Le capot 22 comprend une partie 70 de fixation sur le support 14, annulaire, ainsi qu'une autre partie annulaire 72, coaxiale avec la partie 70, de façon à définir une gorge 74 dans laquelle la paroi annulaire 60 est encastrée. Le capot 22 comprend par ailleurs un siège 76 d'appui du ressort 20, prolongé sur sa périphérie intérieure par une paroi annulaire 78, traversée par la partie 66 et ayant une fonction de centrage de la partie 66 de la valve 16.

De plus, dans cet exemple, l'organe perméable à l'air 28 comprend au moins un canal 80 de passage de liquide. En outre dans cet exemple, le canal 80 de passage de liquide a également une fonction de limitation de débit de liquide, débouchant sur le canal de passage de liquide 24. Plus précisément, la collerette 30 de l'organe 28 comprend sur sa surface annulaire extérieure, une pluralité de gorges 80, représentées notamment sur les figures 2a à 2d, et délimitant, avec le logement 44, des canaux 82 de réduction de débit de liquide. Ces canaux 82 ont un diamètre relativement restreint, de façon à diminuer la pression du liquide lorsque l'utilisateur appuie sur le réservoir. Selon une variante, les gorges 80 pourraient présenter des variations de direction, ou encore une forme hélicoïdale. Selon le nombre et la taille des gorges 80 placées en regard du logement 44, le début de liquide pouvant sortir sera plus ou moins réduit.

L'organe 28 peut prendre par exemple l'une des formes illustrées sur les figures 2a à 2d. Les formes de réduction 80 sont réalisées sur la périphérie extérieure de sa collerette 30, formant un évidement de la périphérie.

Sur les exemples de la figure 2a, l'organe 28 comprend une paroi mince de passage d'air, de forme sensiblement cylindrique ou conique. La paroi comporte en outre, afin d'être rigidifiée, des nervures de renfort 84, correspondant à des augmentations locales de l'épaisseur de la paroi..

Les organes 28 des figures 2b à 2d illustrent d'autres types d'organes 28 sur lesquels la paroi de passage d'air comporte, à la place ou en plus des nervures de renfort 84, une pluralité de reliefs qui permettent d'augmenter la surface d'échange d'air entre l'intérieur et l'extérieur du réservoir 12 sans pour autant augmenter considérablement l'encombrement de l'organe 28. Ces reliefs sont formés dans la paroi de façon qu'elle conserve son épaisseur relativement fine afin de laisser passer l'air. On constate que ces reliefs peuvent permettre en outre de rigidifier l'organe 28 et permettent éventuellement de ne pas utiliser de renforts 84, comme on peut le voir notamment à la figure 2c illustrant une paroi de passage d'air ondulée, ayant une section en forme de trèfle.

Le fonctionnement du dispositif de la figure 1 va à présent être décrit.

Au repos, c'est-à-dire lorsque aucun utilisateur n'appuie pas sur le réservoir 12, la valve 16 est en configuration de blocage du liquide, c'est-à-dire qu'elle est en appui sur la surface 40, du fait de sa fixation permanente sur le support 14, exerçant une contrainte élastique sur la valve, et du fait de la pression effectuée par le ressort 20.

Lorsqu'un utilisateur appuie sur le réservoir 12, il exerce une pression sur le fluide qui s'écoule dans le seul canal autorisant son écoulement, à savoir le canal 82 de passage de liquide et dans cet exemple, de réduction de débit, vu que les parois de l'organe 28 ne peuvent pas être traversées par un liquide. Au cours de son passage dans ce canal 82, dans cet exemple, le fluide voit son débit diminuer, par effet de perte de charge. Le fluide s'écoule ensuite dans le canal 24, puis dans la cavité 48 et dans le canal 50. Sous l'effet de la pression, le fluide soulève la valve 16 qui passe alors en configuration de passage de liquide et peut ainsi s'écouler entre la valve 16 et la surface d'appui 40, afin de passer dans le canal 18 et dans la cavité 68, et ainsi se présenter sous forme de goutte.

Une fois la goutte délivrée, l'utilisateur relâche la pression sur le réservoir 12 déformable, lequel tend à reprendre sa forme initiale, ce qui génère une dépression à l'intérieur du réservoir 12. Cette dépression sera compensée par une reprise d'air extérieur venant du canal de passage d'air 26 à travers l'organe 28 perméable à l'air. On notera que, du fait que le matériau constituant l'organe 28 est non poreux, le passage d'air à travers l'organe 28 est un processus qui prend plusieurs minutes, voire plusieurs heures, et non quelques secondes.

Ainsi, si l'on considère un dispositif d'une contenance de 12 ml, rempli de 10 ml d'une solution ophtalmique et muni d'un organe perméable à l'air comprenant du silicone ayant une perméabilité à l'oxygène de 1,4*10⁻¹³ mol*m⁻¹Pa⁻¹*s⁻¹ (mol par mètre par Pascal et par seconde) et une surface d'échange de 90 mm² et une épaisseur de 0.4 mm, la délivrance de 6 gouttes de solution sous pression atmosphérique, c'est-à-dire de 40*6=240 microlitres de liquide, crée une dépression d'environ 95 mbar qui sera quasiment compensée en 12 heures (plus précisément, environ 90mbar seront compensés au bout de 12 heures).

Vu que la paroi de l'organe 28 n'est pas poreuse, ce temps de reprise d'air dans le réservoir 12 est approximativement le même, que le dispositif soit en position « tête en bas » ou non.

On notera que l'organe 28, étant une pièce à part, peut avoir une forme qui varie en fonction des applications, des temps désirés de reprise d'air et des réductions de débit souhaités. Il est donc possible de fabriquer des lots comprenant des embouts présentant une même valve 16, un même support 14, un même capot 18, mais présentant des organes 28 différents.

On notera que l'invention n'est pas limitée aux modes de réalisation précédemment décrits.

On notera qu'il est particulièrement intéressant d'utiliser un matériau non poreux tel que celui utilisé pour l'organe 28, du fait qu'il est très facile de vérifier que cet organe est fonctionnel. En effet, si l'on avait utilisé un filtre hydrophobe à la place de l'organe 28, il aurait été délicat de tester, après assemblage, que le filtre ne présente pas de fuite.

## Revendications

1. Dispositif de distribution de liquide, comportant:
- un réservoir (12) de liquide, déformable de façon à distribuer du liquide par appui sur le réservoir (12),
- un embout (10) de distribution du liquide monté sur le réservoir (12),
- un canal de passage de liquide (24,50),
- un canal de passage d'air (26) depuis l'extérieur vers l'intérieur du réservoir, le canal de passage d'air (26) étant obturé par un organe (28) réalisé en matériau polymère perméable à l'air, l'organe (28) étant appelé organe (28) perméable à l'air **caractérisé en ce que** le matériau dudit organe (28) perméable à l'air est non poreux.

2. Dispositif selon la revendication précédente, dans lequel l'organe perméable à l'air (28) est configuré pour laisser passer l'air par diffusion de l'air au travers de l'organe (28).

3. Dispositif selon l'une quelconque des revendications précédentes, dans lequel l'organe perméable à l'air (28) comprend en outre au moins un canal (80) de passage de liquide.

4. Dispositif selon la revendication précédente dans lequel le canal (80) de passage de liquide est un canal (80) de limitation de débit de liquide, débouchant sur le canal de passage de liquide (24).

5. Dispositif selon la revendication 3 ou 4, dans lequel le canal (80) de passage de liquide est délimité par une surface annulaire extérieure de l'organe perméable à l'air (28).

6. Dispositif selon l'une quelconque des revendications précédentes, dans lequel l'embout (10) et l'organe (28) ont chacun un axe central, les deux axes étant confondus.

7. Dispositif selon l'une quelconque des revendications précédentes, dans lequel l'organe (28) comporte une paroi, dite paroi de passage d'air, munie d'une pluralité de reliefs.

8. Dispositif selon l'une quelconque des revendications précédentes, dans lequel l'organe (28) comporte des nervures de renfort (84).

9. Dispositif selon l'une quelconque des revendications précédentes, dans lequel l'organe (28) a une forme générale cylindrique ou conique, dont la base comprend une collerette (30) de fixation sur l'embout (10) par exemple par serrage mécanique, la collerette (30) comportant éventuellement des moyens de fixation mécanique qui coopèrent avec des moyens portés par l'embout (10), par exemple par encliquetage.

10. Dispositif selon l'une quelconque des revendications précédentes, dans lequel le matériau polymère comprend du silicone.

11. Dispositif selon l'une quelconque des revendications précédentes, comportant une valve (16) et un support (14) comprenant une surface (40) d'appui de la valve pour bloquer le passage de liquide, le support comprenant le canal (26) de passage d'air, l'organe (28) perméable à l'air étant rapporté sur le support (14).

12. Dispositif selon l'une quelconque des revendications précédentes, dans lequel le temps d'équilibrage des pressions entre la pression interne du réservoir et la pression externe après la distribution de liquide est supérieur à 30 minutes, de préférence 1 heure.

## Patentansprüche

1. Flüssigkeitsverteilungsvorrichtung, die Folgendes aufweist:
- einen Flüssigkeitsbehälter (12), der so verformbar ist, dass er Flüssigkeit durch Drücken auf den Behälter (12) verteilt,
- einen Ansatz (10) zum Verteilen der Flüssigkeit, der auf den Behälter montiert (12) ist,
- einen Flüssigkeitsdurchgangskanal (24, 50),
- einen Luftdurchgangskanal (26) von der Außenseite zur Innenseite des Behälters, wobei der Luftdurchgangskanal (26) durch ein Organ (28) verschlossen ist, das aus einem Luft durchlässigen Polymermaterial hergestellt ist, wobei das Organ (28) Luft durchlässiges Organ (28) genannt wird, **dadurch gekennzeichnet, dass** der Werkstoff des Luft durchlässiges Organs (28) nicht porig ist.

2. Vorrichtung nach dem vorhergehenden Anspruch, bei der das Luft durchlässige Organ (28) konfiguriert ist, um Luft durch Luftdiffusion durch das Organ (28) durchgehen zu lassen.

3. Vorrichtung nach einem der vorhergehenden Ansprüche, bei der das Luft durchlässige Organ (28) ferner mindestens einen Flüssigkeitsdurchgangskanal (80) aufweist.

4. Vorrichtung nach dem vorhergehenden Anspruch, bei der der Flüssigkeitsdurchgangskanal (80) ein Flüssigkeitsdurchflussbegrenzungskanal (80) ist, der auf dem Flüssigkeitsdurchgangskanal (24) mündet.

5. Vorrichtung nach Anspruch 3 oder 4, bei der der Flüssigkeitsdurchgangskanal (80) durch eine ringförmige äußere Oberfläche des Luft durchlässigen Organs (28) abgegrenzt ist.

6. Vorrichtung nach einem der vorhergehenden Ansprüche, bei der der Ansatz (10) und das Organ (28) jeweils eine zentrale Achse haben, wobei die zwei Achsen zusammenfallen.

7. Vorrichtung nach einem der vorhergehenden Ansprüche, bei der das Organ (28) eine Wand, Luftdurchgangswand genannt, die mit mehreren Reliefs versehen ist, aufweist.

8. Vorrichtung nach einem der vorhergehenden Ansprüche, bei der das Organ (28) Verstärkungsrippen (84) aufweist.

9. Vorrichtung nach einem der vorhergehenden Ansprüche, bei der das Organ (28) eine allgemein zylindrische oder konische Form hat, deren Basis einen Kragen (30) zum Befestigen auf dem Ansatz (10) zum Beispiel durch mechanisches Klemmen aufweist, wobei der Kragen (30) eventuell mechanische Befestigungsmittel aufweist, die mit Mitteln, die von dem Ansatz (10) getragen werden, zum Beispiel durch Einrasten zusammenwirken.

10. Vorrichtung nach einem der vorhergehenden Ansprüche, bei der der Polymerwerkstoff Silikon enthält.

11. Vorrichtung nach einem der vorhergehenden Ansprüche, die ein Ventil (16) und einen Träger (14) aufweist, der eine Oberfläche (40) zum Aufliegen des Ventils aufweist, um den Flüssigkeitsdurchgang zu blockieren, wobei der Träger den Luftdurchgangskanal (26) aufweist, wobei das Luft durchlässige Organ (28) auf dem Träger (14) angebaut ist.

12. Vorrichtung nach einem der vorhergehenden Ansprüche, bei der die Ausgleichszeit der Drücke zwischen dem internen Druck des Behälters und dem externen Druck nach dem Flüssigkeitsverteilen größer ist als 30 Minuten, vorzugsweise 1 Stunde.

## Claims

1. Device for dispensing liquid, comprising:
- a liquid reservoir (12) which can be deformed so as to dispense liquid by pressing on the reservoir (12),
- a liquid-dispensing end piece (10) fitted on the reservoir (12),
- a channel (24, 50) for the passage of liquid,
- a channel (26) for the passage of air from the outside to the inside of the reservoir, the channel (26) for the passage of air being closed off by a member (28) made of an air-permeable polymeric material, the member (28) being called the air-permeable member (28), **characterised in that** the material of the air-permeable member (28) is non-porous.

2. Device according to the preceding claim, wherein the air-permeable member (28) is configured to allow air to pass by diffusion across the air-permeable member (28).

3. Device according to any of the preceding claims, wherein the air-permeable member (28) further comprises at least one channel (80) for the passage of liquid.

4. Device according to the preceding claim, wherein the channel (80) for the passage of liquid is a liquid flow limitation channel (80) opening to the channel (24) for the passage of liquid.

5. Device according to claim 3 or 4, wherein the channel (80) for the passage of liquid is bounded by an outer annular surface of the air-permeable member (28).

6. Device according to any of the preceding claims, wherein the end piece (10) and the member (28) each has a central axis, the two axes being colinear.

7. Device according to any of the preceding claims, wherein the member (28) comprises a so-called air passage wall, equipped with a plurality of reliefs.

8. Device according to any of the preceding claims, wherein the member (28) comprises stiffening ribs (84).

9. Device according to any of the preceding claims, wherein the member (28) has a generally cylindrical or conical shape, with a base comprising a collar (30) for fastening to the end piece (10) for example by mechanical fastening, the collar (30) possibly comprising mechanical fastening means which cooperate with means on the end piece (10), for example by snap fastening.

10. Device according to any of the preceding claims, wherein the polymeric material includes silicone.

11. Device according to any of the preceding claims, including a valve (16) and a support (14) comprising a valve bearing surface (40) to block the passage of liquid, the support comprising the channel (26) for the passage of air, the air-permeable member (28) being attached to the support (14).

12. Device according to any of the preceding claims, wherein the time to balance the pressures inside and outside the reservoir after dispensing liquid is greater than 30 minutes, preferably 1 hour.
